# EUROPEAN PATENT APPLICATION

(11) **EP 3 888 678 A1**
(43) Date of publication of application: **06.10.2021**
(21) Application number: 19890307.2
(22) Date of filing: 26.11.2019
(51) Int. Cl.: A61K 39/395, A61K 9/08, A61K 47/00, A61P 35/00

(54) **ANTI-PD-L1 ANTIBODY PREPARATION**

(30) Priority: 29.11.2018 CN 201811441890
(71) Applicant: Harbour Biomed Therapeutics Limited, Hong Kong (CN); Sichuan Kelun-Biotech Biopharmaceutical Co., Ltd., Chengdu, Sichuan 611138 (CN)
(72) Inventor: XIAO, Liang, Chengdu, Sichuan 611138 (CN); DENG, Liping, Chengdu, Sichuan 611138 (CN); HUANG, Haiyu, Chengdu, Sichuan 611138 (CN); LIU, Qigang, Chengdu, Sichuan 611138 (CN); LIU, Liping, Chengdu, Sichuan 611138 (CN); GUO, Dalton, Chengdu, Sichuan 611138 (CN); XUE, Tongtong, Chengdu, Sichuan 611138 (CN); WANG, Jingyi, Chengdu, Sichuan 611138 (CN)
(74) Representative: Lasar, Andrea Gisela
(86) International application number: PCT/CN2019/121069
(87) International publication number: WO 2020/108497

(57) **Abstract**

Provided is an anti-PD-Ll antibody preparation, comprising an anti-PD-Ll antibody, a buffer solution and sodium chloride, without added sugars and/or sugar alcohols. Also provided is the use of the anti-PD-Ll antibody preparation in the preparation of drugs for preventing and/or treating and/or adjuvant treating and/or diagnosing a tumor or anemia.

SEQUENCE LISTING

<110>  HARBOUR BIOMED THERAPEUTICS LIMITED
       SICHUAN KELUN-BIOTECH BIOPHARMACEUTICAL CO., LTD.

<120>  ANTI-PD-L1 ANTIBODY PREPARATION

<130>  H10268WOEP/LA

<140>  19890307.2
<141>  2019-11-26

<150>  CN201811441890.4
<151>  2018-11-29

<160>  10

<170>  PatentIn version 3.5

<210>  1
<211>  8
<212>  PRT
<213>  Artificial Sequence

<400>  1

Gly Phe Ser Leu Ser Asn Tyr Asp
1               5


<210>  2
<211>  7
<212>  PRT
<213>  Artificial Sequence

<400>  2

Ile Trp Thr Gly Gly Ala Thr
1               5


<210>  3
<211>  14
<212>  PRT
<213>  Artificial Sequence

<400>  3

Val Arg Asp Ser Asn Tyr Arg Tyr Asp Glu Pro Phe Thr Tyr
1               5                   10


<210>  4
<211>  6
<212>  PRT
<213>  Artificial Sequence

<400>  4

Gln Ser Ile Gly Thr Asn
1               5

<210>  5
<211>  3
<212>  PRT
<213>  Artificial Sequence

<400>  5

Tyr Ala Ser
1


<210>  6
<211>  9
<212>  PRT
<213>  Artificial Sequence

<400>  6

Gln Gln Ser Asn Ser Trp Pro Tyr Thr
1               5


<210>  7
<211>  120
<212>  PRT
<213>  Artificial Sequence

<400>  7

Gln Val Gln Leu Gln Glu Ser Gly Pro Gly Leu Val Lys Pro Ser Glu
1               5                   10                  15

Thr Leu Ser Ile Thr Cys Thr Val Ser Gly Phe Ser Leu Ser Asn Tyr
            20                  25                  30

Asp Ile Ser Trp Ile Arg Gln Pro Pro Gly Lys Gly Leu Glu Trp Leu
        35                  40                  45

Gly Val Ile Trp Thr Gly Gly Ala Thr Asn Tyr Asn Pro Ala Leu Lys
    50                  55                  60

Ser Arg Leu Thr Ile Ser Arg Asp Asn Ser Lys Asn Gln Val Ser Leu
65                  70                  75                  80

Lys Met Ser Ser Val Thr Ala Ala Asp Thr Ala Val Tyr Tyr Cys Val
                85                  90                  95

Arg Asp Ser Asn Tyr Arg Tyr Asp Glu Pro Phe Thr Tyr Trp Gly Gln
            100                 105                 110

Gly Thr Leu Val Thr Val Ser Ser
        115                 120


<210>  8
<211>  107

<212>  PRT
<213>  Artificial Sequence

<400>  8

Glu Ile Val Leu Thr Gln Ser Pro Asp Thr Leu Ser Val Thr Pro Lys
1               5                   10                  15

Glu Lys Val Thr Leu Thr Cys Arg Ala Ser Gln Ser Ile Gly Thr Asn
            20                  25                  30

Ile His Trp Phe Gln Gln Lys Pro Gly Gln Ser Pro Lys Leu Leu Ile
        35                  40                  45

Lys Tyr Ala Ser Glu Ser Ile Ser Gly Val Pro Ser Arg Phe Ser Gly
    50                  55                  60

Ser Gly Ser Gly Thr Asp Phe Thr Leu Thr Ile Asn Ser Val Glu Ala
65                  70                  75                  80

Glu Asp Ala Ala Thr Tyr Tyr Cys Gln Gln Ser Asn Ser Trp Pro Tyr
                85                  90                  95

Thr Phe Gly Gln Gly Thr Lys Leu Glu Ile Lys
            100                 105


<210>  9
<211>  450
<212>  PRT
<213>  Artificial Sequence

<400>  9

Gln Val Gln Leu Gln Glu Ser Gly Pro Gly Leu Val Lys Pro Ser Glu
1               5                   10                  15

Thr Leu Ser Ile Thr Cys Thr Val Ser Gly Phe Ser Leu Ser Asn Tyr
            20                  25                  30

Asp Ile Ser Trp Ile Arg Gln Pro Pro Gly Lys Gly Leu Glu Trp Leu
        35                  40                  45

Gly Val Ile Trp Thr Gly Gly Ala Thr Asn Tyr Asn Pro Ala Leu Lys
    50                  55                  60

Ser Arg Leu Thr Ile Ser Arg Asp Asn Ser Lys Asn Gln Val Ser Leu
65                  70                  75                  80

Lys Met Ser Ser Val Thr Ala Ala Asp Thr Ala Val Tyr Tyr Cys Val
                85                  90                  95

Arg Asp Ser Asn Tyr Arg Tyr Asp Glu Pro Phe Thr Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Ala Ala Gly Ala
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Asp Glu Leu Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly Lys
    450

<210>  10
<211>  214
<212>  PRT
<213>  Artificial Sequence

<400>  10

Glu Ile Val Leu Thr Gln Ser Pro Asp Thr Leu Ser Val Thr Pro Lys
1               5                   10                  15

Glu Lys Val Thr Leu Thr Cys Arg Ala Ser Gln Ser Ile Gly Thr Asn
            20                  25                  30

Ile His Trp Phe Gln Gln Lys Pro Gly Gln Ser Pro Lys Leu Leu Ile
        35                  40                  45

Lys Tyr Ala Ser Glu Ser Ile Ser Gly Val Pro Ser Arg Phe Ser Gly
    50                  55                  60

Ser Gly Ser Gly Thr Asp Phe Thr Leu Thr Ile Asn Ser Val Glu Ala
65                  70                  75                  80

Glu Asp Ala Ala Thr Tyr Tyr Cys Gln Gln Ser Asn Ser Trp Pro Tyr
                85                  90                  95

Thr Phe Gly Gln Gly Thr Lys Leu Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

## Description

### TECHNICAL FIELD

The invention relates to the field of therapeutic antibodies. More specifically, the invention relates to an anti-PD-L1 antibody preparation and medical use thereof.

### BACKGROUND

PD-1 (Programmed Death 1), with the full name of programmed death receptor 1, is a type I transmembrane glycoprotein belonging to members of the CD28 family. PD-L1 (Programmed Cell Death-Ligand 1), with the full name of programmed death receptor-ligand 1, is a type I transmembrane glycoprotein with a size of 40KDa.

Under normal physiological conditions, after an antigen enters the body, it is processed by antigen presenting cells (APCs) and connected with MHC. TCR molecules on the surface of T cells specifically recognize antigen peptides presented by APCs, and at the same time recognize MHC molecules that form complexes with antigen peptides. In addition, numerous immunomodulatory molecules on the surface of T cells participate in the response to generate costimulatory signals, promoting T cell activation and exerting immunological effects. PD-1 is expressed on the surface of activated T cells, B cells, NK cells, monocytes and DCs, which interacts with PD-L1 and PD-L2, etc. on the surface of APC cells to transmit inhibitory signals, inhibit the activation of T cells, and maintain the body's immune homeostasis. High expression of PD-L1 on the surface of tumor cells in combination with high expression of PD-1 of tumor-infiltrating T lymphocytes lead to continuous activation of the PD-1 pathway, thereby inhibiting T cell activation and resulting in immune escape of tumors. Therefore, blocking the binding of PD-1/PD-L1 has become one of the important strategies in the field of tumor immunotherapy. PD-L1 antibodies can block the binding of PD-L1 on tumors and PD-1 on T cells to eliminate this immunosuppressive effect, so that T cells are reactivated to recognize and kill tumor cells.

WO2017/148424 discloses new anti-PD-L1 monoclonal antibodies, which can effectively block the binding of PD-1 to PD-L1, and show better therapeutic effects on tumors or anemia. Nevertheless, the use of PD-L1 antibody in clinical treatment needs to solve the problems of physical and chemical properties suitable for administration and stability during storage. Antibody preparation is the final form of antibody in storage, transportation and clinical treatment, which needs to take into account its physical and chemical properties, physiological properties and stability within the validity period, such as protein content, pH, osmotic pressure, insoluble particles, purity, aggregates, binding activity, etc. As different antibodies have their own characteristics in terms of physical and chemical properties, and their stability varies greatly, it is difficult to obtain a stable and universal liquid formulation when preparing preparations. Therefore, for each innovatively developed antibody, it is necessary to conduct in-depth research to find a suitable formulation to obtain an antibody preparation with good physiological compatibility for the convenience of drug storage and clinical application.

Therefore, for anti-PD-Ll antibodies, it is necessary to develop appropriate formulations to obtain antibody preparations that satisfy the requirements for physiological compatibility and stability.

### SUMMARY

After a large number of experiments and elaborate explorations, the inventors of the application unexpectedly discovered that the liquid preparation composed of anti-PD-Ll antibody and a specific formulation has excellent physiological compatibility and stability, and further developed high concentration of antibody preparations. The high-concentration protein preparation overcomes the problems of poor stability, high viscosity and easy turbidity. Accordingly, the present application discloses a liquid preparation that is particularly advantageous for long-term storage and clinical use of drugs, thereby completing the invention.

### Liquid preparation

Accordingly, in one aspect, the invention provides a liquid preparation comprising:
(1) an anti-PD-Ll antibody at a concentration of about 10 mg/ml to about 150 mg/ml; wherein the anti-PD-Ll antibody comprises heavy chain CDRs as shown in SEQ ID NOs: 1-3 respectively, and light chain CDRs as shown in SEQ ID NOs: 4-6, respectively;
(2) a buffer at a concentration of about 5 mM to about 40 mM;
(3) sodium chloride at a concentration of about 80 mM to about 160 mM;
wherein the liquid preparation has a pH of about 5.0 to about 7.0; and the liquid preparation has no added sugar and/or sugar alcohol;
and the liquid preparation has an osmotic pressure comparable to that of human blood.

The anti-PD-Ll antibody liquid preparation disclosed herein is characterized in the absence of added sugars and/or sugar alcohols, and thus can also be referred to as sugar-free antibody preparation herein.

In certain preferred embodiments, the liquid preparation does not contain a sugar and/or a sugar alcohol selected from the group consisting of sucrose, glucose, trehalose, mannitol and sorbitol.

In certain preferred embodiments, the anti-PD-Ll antibody has a heavy chain variable region as shown in SEQ ID NO: 7 and a light chain variable region as shown in SEQ ID NO: 8. In certain preferred embodiments, the anti-PD-Ll antibody has a heavy chain as shown in SEQ ID NO: 9 and a light chain as shown in SEQ ID NO: 10.

In another preferred embodiment, the concentration of the anti-PD-Ll antibody is 10 mg/ml to 30 mg/ml.

In another preferred embodiment, the concentration of the anti-PD-Ll antibody is 10 mg/ml to 120 mg/ml, preferably 30 mg/ml to 70 mg/ml.

In certain preferred embodiments, the concentration of the anti-PD-Ll antibody is about 15 mg/ml to about 25 mg/ml. In certain preferred embodiments, the concentration of the anti-PD-Ll antibody is about 20 mg/ml.

In certain preferred embodiments, the concentration of the anti-PD-Ll antibody is about 50 mg/ml to about 70 mg/ml. In certain preferred embodiments, the concentration of the anti-PD-Ll antibody is about 60 mg/ml.

In certain preferred embodiments, the concentration of the anti-PD-Ll antibody is about 80 mg/ml to about 150 mg/ml. In certain preferred embodiments, the concentration of the anti-PD-Ll antibody is about 90 mg/ml, about 100 mg/ml, about 110 mg/ml, or about 120 mg/ml.

In certain preferred embodiments, the concentration of the sodium chloride is about 100 mM to about 160 mM. In certain preferred embodiments, the concentration of the sodium chloride is about 105 mM, about 110 mM, about 115 mM, about 120 mM, about 130 mM, about 135 mM, about 140 mM, about 145 mM, about 150 mM, or about 155 mM. In certain more preferred embodiments, the concentration of the sodium chloride is about 140 mM.

In certain preferred embodiments, the buffer is selected from: citric acid-citrate buffer, histidine buffer, or acetic acid-acetate buffer. In certain preferred embodiments, the histidine is present alone or in the form of histidine hydrochloride or histidine acetate in the histidine buffer.

In certain preferred embodiments, the buffer is a histidine buffer. In certain preferred embodiments, the histidine is present alone or in the form of histidine-hydrochloride or histidine-acetate in the histidine buffer.

In certain preferred embodiments, the concentration of the buffer is about 10 mM to about 20 mM. In certain preferred embodiments, the concentration of the buffer is about 20 mM. In certain preferred embodiments, the liquid preparation contains a histidine buffer at a concentration of about 20 mM, preferably the histidine is present alone or in the form of histidine hydrochloride or histidine acetate in the histidine buffer.

In another preferred embodiment, the buffer is 20 mM of histidine-histidine hydrochloride.

In the invention, the pH of the liquid preparation is adjusted by a buffer, whose pH may be further adjusted using a pH adjusting agent (e.g., hydrochloric acid, acetic acid, phosphoric acid, etc.). In certain preferred embodiments, the liquid preparation has a pH of about 5.2 to about 6.8. In certain preferred embodiments, the liquid preparation has a pH of about 5.6 to about 6.8. In certain preferred embodiments, the liquid preparation has a pH of about 5.6 to about 6.0. In certain exemplary embodiments, the pH of the liquid preparation is about 5.0, about 5.2, about 5.6, about 5.8, about 6.0, about 6.4, about 6.8, or about 7.0. In certain exemplary embodiments, the pH of the liquid preparation is 5.8±0.1. In certain exemplary embodiments, the pH of the liquid preparation is about 5.8.

In certain preferred embodiments, the liquid preparation has an osmolality of about 260 mOsmol/kg to about 380 mOsmol/kg. In certain preferred embodiments, the liquid preparation has an osmolality of about 280 mOsmol/kg to about 380 mOsmol/kg (e.g., 280 mOsmol/kg to about 360 mOsmol/kg). In certain preferred embodiments, the liquid preparation has an osmolality of about 280 mOsmol/kg to about 330 mOsmol/kg. In certain preferred embodiments, the liquid preparation has an osmotic pressure that is substantially the same as that of human blood.

In another preferred example, the osmotic pressure comparable to human blood is an osmolality of about 280 mOsmol/kg to about 400 mOsmol/kg, preferably an osmolality of 290 mOsmol/kg to about 320 mOsmol/kg, more preferably an osmolality of 295 mOsmol/kg to about 315 mOsmol/kg.

In certain preferred embodiments, the liquid preparation further comprises a surfactant. In certain preferred embodiments, the surfactant is polysorbate 80/polysorbate 20 or poloxamer 188. In certain preferred embodiments, the surfactant is polysorbate 80.

In certain preferred embodiments, the concentration of the surfactant is about 0.01% w/v to about 0.02% w/v. In certain preferred embodiments, the concentration of the surfactant is about 0.02% w/v. In certain preferred embodiments, the liquid preparation comprises polysorbate 80 at a concentration of about 0.02% w/v.

In certain preferred embodiments, the liquid preparation comprises:
(1) the anti-PD-Ll antibody at a concentration of about 20 mg/ml, about 60 mg/ml, about 90 mg/ml or about 120 mg/ml;
(2) a buffer at a concentration of about 20 mM; the buffer is selected from histidine buffer, citric acid-citrate buffer and acetic acid-acetate buffer;
(3) sodium chloride at a concentration of about 140 mM;
wherein the liquid preparation has a pH of about 5.2 to about 6.8 (e.g., a pH of about 5.6 to about 6.8, such as a pH of about 5.6 to about 6.0, such as a pH of about 5.8); and the liquid preparation does not contain sugars and/or sugar alcohols.

In certain preferred embodiments, the liquid preparation of the invention can be stable at 5°C±3°C and can be stored for at least about 2 weeks, at least about 1 month, at least about 2 months, at least about 3 months, at least about 6 months, at least about 9 months, at least about 1 year, or at least about 2 years. In certain preferred embodiments, the liquid preparation of the invention is stable at about 40°C and can be stored for at least about 2 weeks, at least about 1 month, at least about 2 months, at least about 3 months, at least about 6 months, at least about 9 months, at least about 1 year, or at least about 2 years. In certain preferred embodiments, the liquid preparation of the invention is stable at room temperature (e.g., about 25°C) and can be stored for at least about 2 weeks, at least about 1 month, at least about 2 months, at least about 3 months, at least About 6 months, at least about 9 months, at least about 1 year, or at least about 2 years.

In certain exemplary embodiments, the liquid preparation comprises: the anti-PD-Ll antibody at a concentration of 20 mg/ml, sodium chloride at a concentration of 140 mM, histidine-hydrochloride buffer at a concentration of 20 mM, and polysorbate 80 at a concentration of 0.02% w/v; and the pH of the liquid preparation is 5.8. In such embodiments, the liquid preparation is stable at 40°C for at least 28 days and stable at 5°C ± 3°C for at least 12 months.

In certain exemplary embodiments, the liquid preparation comprises: the anti-PD-Ll antibody at a concentration of 20 mg/ml, sodium chloride at a concentration of 140 mM, histidine-hydrochloride buffer at a concentration of 20 mM, and polysorbate 80 at a concentration of 0.02% w/v; and the pH of the liquid preparation is 5.0. In such embodiments, the liquid preparation is stable at 40°C for at least 28 days.

In certain exemplary embodiments, the liquid preparation comprises: the anti-PD-Ll antibody at a concentration of 20 mg/ml, sodium chloride at a concentration of 140 mM, histidine-hydrochloride buffer at a concentration of 20 mM, and polysorbate 80 at a concentration of 0.02% w/v; and the pH of the liquid preparation is 7.0. In such embodiments, the liquid preparation is stable at 40°C for at least 28 days.

In certain exemplary embodiments, the liquid preparation comprises: the anti-PD-Ll antibody at a concentration of 20 mg/ml, sodium chloride at a concentration of 140 mM, histidine-acetate buffer at a concentration of 20 mM, and polysorbate 80 at a concentration of 0.02% w/v; and the pH of the liquid preparation is 5.8. In such embodiments, the liquid preparation is stable at 40°C for at least 28 days.

In certain exemplary embodiments, the liquid preparation comprises: the anti-PD-Ll antibody at a concentration of 20 mg/ml, sodium chloride at a concentration of 140 mM, acetic acid-sodium acetate buffer at a concentration of 20 mM, and polysorbate 80 at a concentration of 0.02% w/v; and the pH of the liquid preparation is 5.8. In such embodiments, the liquid preparation is stable at 40°C for at least 28 days.

In certain exemplary embodiments, the liquid preparation comprises: the anti-PD-Ll antibody at a concentration of 20 mg/ml, sodium chloride at a concentration of 140 mM, citric acid-sodium citrate buffer at a concentration of 20 mM, and polysorbate 80 at a concentration of 0.02% w/v; and the pH of the liquid preparation is 5.8. In such embodiments, the liquid preparation is stable at 40°C for at least 28 days.

In certain exemplary embodiments, the liquid preparation comprises: the anti-PD-Ll antibody at a concentration of 20 mg/ml, sodium chloride at a concentration of 140 mM, histidine buffer present in the form of histidine alone at a concentration of 20 mM, and polysorbate 80 at a concentration of 0.02% w/v; and the pH of the liquid preparation is 5.8. In such embodiments, the liquid preparation is stable at 40°C for at least 28 days, stable at 25°C for at least 6 months, and stable at 5°C ± 3°C for at least 36 months.

In certain exemplary embodiments, the liquid preparation comprises: the anti-PD-Ll antibody at a concentration of 20 mg/ml, sodium chloride at a concentration of 140 mM, histidine-histidine hydrochloride buffer at a concentration of 20 mM, and polysorbate 80 at a concentration of 0.02% w/v; and the pH of the liquid preparation is 5.8. In such embodiments, the liquid preparation is stable at 40°C for at least 28 days, stable at 25°C for at least 6 months, and stable at 5°C ± 3°C for at least 36 months.

In certain exemplary embodiments, the liquid preparation comprises: the anti-PD-Ll antibody at a concentration of 60 mg/ml, sodium chloride at a concentration of 140 mM, histidine buffer present in the form of histidine alone at a concentration of 10 mM, and polysorbate 80 at a concentration of 0.02% w/v; and the pH of the liquid preparation is 5.7. In such embodiments, the liquid preparation is stable at 40°C for at least 14 days.

In certain exemplary embodiments, the liquid preparation comprises: the anti-PD-Ll antibody at a concentration of 60 mg/ml, sodium chloride at a concentration of 140 mM, histidine-histidine hydrochloride buffer at a concentration of 10 mM, and polysorbate 80 at a concentration of 0.02% w/v; and the pH of the liquid preparation is 5.7. In such embodiments, the liquid preparation is stable at 40°C for at least 14 days.

In certain exemplary embodiments, the liquid preparation comprises: the anti-PD-Ll antibody at a concentration of 60 mg/ml, sodium chloride at a concentration of 140 mM, histidine buffer present in the form of histidine alone at a concentration of 20 mM, and polysorbate 80 at a concentration of 0.02% w/v; and the pH of the liquid preparation is 5.8. The liquid preparation can be stable at 5°C for at least 6 months.

In certain exemplary embodiments, the liquid preparation comprises: the anti-PD-Ll antibody at a concentration of 60 mg/ml, sodium chloride at a concentration of 140 mM, histidine-histidine hydrochloride buffer at a concentration of 20 mM, and polysorbate 80 at a concentration of 0.02% w/v; and the pH of the liquid preparation is 5.8. The liquid preparation can be stable at 5°C for at least 6 months.

In certain exemplary embodiments, the liquid preparation comprises: the anti-PD-Ll antibody at a concentration of 90 mg/ml, sodium chloride at a concentration of 140 mM, histidine buffer present in the form of histidine alone at a concentration of 20 mM, and polysorbate 80 at a concentration of 0.02% w/v; and the pH of the liquid preparation is 5.8. In such embodiments, the liquid preparation is stable at 40°C for at least 13 days and stable at 25°C ± 2°C for at least 2 months.

In certain exemplary embodiments, the liquid preparation comprises: the anti-PD-Ll antibody at a concentration of 90 mg/ml, sodium chloride at a concentration of 140 mM, histidine buffer present in the form of histidine alone at a concentration of 20 mM, and polysorbate 80 at a concentration of 0.02% w/v; and the pH of the liquid preparation is 5.8. In such embodiments, the liquid preparation is stable at 40°C for at least 13 days and stable at 25°C ± 2°C for at least 2 months.

In certain exemplary embodiments, the liquid preparation comprises: the anti-PD-Ll antibody at a concentration of 120 mg/ml, sodium chloride at a concentration of 140 mM, histidine-hydrochloride buffer at a concentration of 20 mM, and polysorbate 80 at a concentration of 0.02% w/v; and the pH of the liquid preparation is 5.8. In such embodiments, the liquid preparation is stable at 40°C for at least 13 days and stable at 25°C ± 2°C for at least 2 months.

In certain exemplary embodiments, the liquid preparation comprises: the anti-PD-Ll antibody at a concentration of 120 mg/ml, sodium chloride at a concentration of 140 mM, histidine-hydrochloride buffer at a concentration of 20 mM, and polysorbate 80 at a concentration is 0.02% w/v; and the pH of the liquid preparation is 5.8. In such embodiments, the liquid preparation is stable at 40°C for at least 13 days and stable at 25°C ± 2°C for at least 2 months.

In certain exemplary embodiments, the liquid preparation comprises: the anti-PD-Ll antibody at a concentration of 20 mg/ml, sodium chloride at a concentration of 100 mM, and citric acid-sodium citrate buffer at a concentration of 20 mM, and the pH of the liquid preparation is 5.6. In such embodiments, the liquid preparation sample has a Tm value greater than 65°C and is stable at 40°C for at least 3 days.

In certain exemplary embodiments, the liquid preparation comprises: the anti-PD-Ll antibody at a concentration of 20 mg/ml, sodium chloride at a concentration of 100 mM, and citric acid-sodium citrate buffer at a concentration of 20 mM, and the pH of the liquid preparation is 6.0. In such embodiments, the liquid preparation sample has a Tm value greater than 65°C and is stable at 40°C for at least 3 days.

In certain exemplary embodiments, the liquid preparation comprises: the anti-PD-Ll antibody at a concentration of 20 mg/ml, sodium chloride at a concentration of 100 mM, and citric acid-sodium citrate buffer at a concentration of 20 mM, and the pH of the liquid preparation is 6.8. In such embodiments, the liquid preparation sample has a Tm value greater than 65°C and is stable at 40°C for at least 3 days.

In another preferred embodiment, the specification of the liquid preparation ranges from 150 mg (10 ml)/vial to 2000 mg (10 ml)/vial.

In another preferred example, the specification of the liquid preparation is selected from the group consisting of: 200 mg (10ml)/vial, 600 mg (10ml)/vial, 900 mg (10ml)/vial, 1200 mg (10ml)/vial or 1500 mg (10ml) /vial. The anti-PD-Ll antibody liquid preparation of the invention can be prepared by combining various components at predetermined concentrations using methods known in the art. For example, the anti-PD-Ll antibody can be dialyzed into a solution comprising other components in the liquid preparation and adjusted to a desired concentration. The preparation is sterilized by filtration with a 0.22 µm pore size filter. The prepared preparations are packaged for ease of use. The packaging material can be a glass vial (e.g., a penicillin vial), a metal alloy container, a pre-filled syringe or a pen-type syringe.

The anti-PD-Ll antibody liquid preparation of the invention can be contained in any container suitable for storing drugs and other pharmaceutical compositions. For example, the liquid preparation of the invention may be contained in a sealed and sterilized plastic container, a metal alloy container or a glass container with a certain volume, such as an ampoule, a penicillin vial or a syringe. Accordingly, in certain preferred embodiments, the liquid preparation of the invention is contained in a glass vial (e.g., an ampoule or a penicillin vial), a syringe or a micro infusion set. In certain exemplary embodiments, the liquid preparation of the invention is contained in a glass vial (e.g., an ampoule or a penicillin vial).

Accordingly, in another aspect, the invention also provides a product comprising a container filled with the liquid preparation of the invention. In certain preferred embodiments, a suitable container includes, for example, a plastic container, a metal alloy container, or a glass container, such as an ampoule, a penicillin vial, or a syringe. In certain preferred embodiments, the product of the invention comprises a container filled with the liquid preparation of the invention, and the suitable container is a glass vial, metal alloy container or pre-filled syringe. In certain preferred embodiments, the product may further include a package insert printed with instructions for use.

The anti-PD-Ll antibody liquid preparation of the invention can be administered to a subject through a parenteral route, such as injection (for example, subcutaneously, intravenously, intramuscularly, intraperitoneally, etc.), or transdermal, mucosal, nasal, respiratory and/or oral administration. In certain preferred embodiments, the liquid preparation is administered to a subject (such as a human) by intravenous injection or subcutaneous injection.

### Therapeutic use of the liquid preparation

The anti-PD-Ll antibody liquid preparation of the invention can be used to prevent and/or treat a tumor or anemia by inhibiting and/or blocking intracellular signaling mediated by PD-1 binding to PD-L1.

Accordingly, in another aspect, the invention also relates to the use of the liquid preparation of the invention in the preparation of a medicament for:
(a) prevention and/or treatment of a tumor or anemia;
(b) adjuvant treatment of a tumor or anemia;
(c) diagnosis of a tumor or anemia;
(d) any combination of (a)-(c).

In certain embodiments, the liquid preparation of the invention is administered separately, in combination, simultaneously, or sequentially with an additional pharmaceutically active agent for the prevention and/or treatment and/or adjuvant treatment of a tumor or anemia. In certain embodiments, the additional pharmaceutically active agent is, for example, a chemotherapeutic drug. In certain preferred embodiments, the tumor is selected from breast cancer, lung cancer such as non-small cell lung cancer and lung squamous cell carcinoma, liver cancer, gastric cancer, bowel cancer such as colon cancer or rectal cancer, esophageal cancer, ovarian cancer, cervical cancer, kidney cancer, prostate cancer, bladder cancer, pancreatic cancer, Merkel cell carcinoma, cholangiocarcinoma, nasopharyngeal carcinoma, and head and neck squamous cell carcinoma, glioma, melanoma, leukemia and lymphoma.

In certain preferred embodiments, the lymphoma is Hodgkin's lymphoma or non-Hodgkin's lymphoma. In certain preferred embodiments, the non-Hodgkin's lymphoma is one or more of peripheral T-cell lymphoma, angioimmunoblastic T-cell lymphoma, NK/T-cell lymphoma, and B-cell non-Hodgkin's lymphoma.

### Definition of terms

In the invention, unless otherwise specified, the scientific and technical terms used herein have the meanings as commonly understood by those skilled in the art. In addition, the pharmacy, biochemistry, nucleic acid chemistry, immunology laboratory and other operating procedures used herein are all routine procedures widely used in the corresponding fields. In the meantime, in order to better understand the invention, definitions and explanations of related terms are provided below.

As used herein, the term "antibody" (Ab) shall include, but is not limited to, an immunoglobulin that specifically binds to an antigen and comprises at least two heavy (H) chains and two light (L) chains interconnected by disulfide bonds. Each heavy chain comprises a heavy chain variable region (abbreviated as VH herein) and a heavy chain constant region. The heavy chain constant region comprises three constant domains, CHI, CH2 and CH3. Each light chain comprises a light chain variable region (abbreviated as VL herein) and a light chain constant region. The light chain constant region comprises a constant domain CL. The VH and VL regions can further be subdivided into regions with hypervariability (called complementarity determining regions (CDRs)), interspersed with more conservative regions (called framework regions (FRs)). Each VH and VL is composed of 3 CDRs and 4 FRs arranged in the following order from the amino terminus to the carboxy terminus: FR1, CDR1, FR2, CDR2, FR3, CDR3, and FR4. The variable regions of each heavy chain/light chain pair (VH and VL) form an antigen binding site separately. Based on the amino acid sequence of the constant region and the amino acid sequence of the framework region of the variable region, the heavy chain can be classified as µ, δ, γ, α, or ε, and the isotype of the antibody is defined as IgM, IgD, IgG, IgA and IgE, respectively, and "light chain" is divided into two strict types: kappa (κ) and lambda (λ).

The term "antibody" includes, for example, naturally occurring and non-naturally occurring antibodies; monoclonal and polyclonal antibodies; chimeric and humanized antibodies; human or non-human antibodies; and fully synthetic antibodies. Non-human antibodies can be humanized by recombinant methods to reduce their immunogenicity in humans. Unless specifically stated otherwise, the "antibody" used herein should be understood to comprise an intact antibody molecule, i.e., immunoglobulin IgG.

The term "anti-PD-Ll antibody" refers to an antibody capable of specifically binding PD-L1. The term "specifically binding" means that the antibody and the antigen form a relatively stable complex under physiological conditions. Specific binding can be characterized by an equilibrium dissociation constant (K_{D}) of about 1×10⁻⁹ M or less. Methods for determining whether two molecules specifically bind each other are well-known in the art, such as bioluminescence interferometry or surface plasmon resonance technology (such as Biacore). Anti-PD-Ll antibodies (intact antibody molecules) suitable for use in the liquid preparations disclosed herein are presented in PCT Patent Publication No. WO2017/148424, which is incorporated herein by reference in its entirety. In certain preferred embodiments, the anti-PD-Ll antibody described herein comprises the heavy chain CDRs as shown in SEQ ID NOs: 1-3 respectively, and the light chain CDRs as shown in SEQ ID NOs: 4-6, respectively. In certain preferred embodiments, the anti-PD-Ll antibody described herein has a heavy chain variable region as shown in SEQ ID NO: 7 and a light chain variable region as shown in SEQ ID NO: 8. In certain preferred embodiments, the anti-PD-Ll antibody described herein has a heavy chain as shown in SEQ ID NO: 9 and a light chain as shown in SEQ ID NO: 10. Lys at the end of the heavy chain is easily missing, which, however, does not affect biological activity. See Dick, L. W. et al., Biotechnol. Bioeng., 100: 1132-1143.

As used herein, the term "programmed death-1 (PD-1)" refers to an immunosuppressive receptor belonging to the CD28 family. PD-1 is mainly expressed on previously activated T cells *in vivo* and binds to two ligands, PD-L1 and PD-L2. The complete hPD-1 sequence can be found under GenBank accession number U64863. Accordingly, the term "programmed death ligand-1 (PD-L1)" is one of the two cell-surface glycoprotein ligands of PD-1 (the other is PD-L2), which down regulates T cell activation and cytokine secretion when binding to PD-1. The term "PD-L1" as used herein includes human PD-L1 (h PD-L1), variants, isoforms, and species homologs of h PD-L1, as well as analogs having at least one epitope in common with h PD-L1. The complete h PD-L1 sequence can be found under GenBank accession number Q9NZQ7.

As used herein, the term "liquid preparation" represents a combination of at least one active ingredient (for example, an antibody, which is capable of exerting biological activity in a subject) and at least one inactive ingredient, and the inactive ingredient should be suitable for administration to a subject upon combined with the active ingredient. Typically, the liquid preparation does not comprise ingredients with unacceptable toxicity to the subject to whom the preparation is to be administrated. Herein, the subject may include mammals, preferably humans.

As used herein, the expression "stability" of an antibody means that an antibody substantially retains its physical stability and/or chemical stability and/or biological activity after storage. The storage period is generally selected based on the intended shelf life of the preparation. Analytical techniques for measuring antibody stability are well known in the art, and stability can be determined at a selected temperature for a selected time period (for example, the stability of the antibody is tested during storage at 40°C for at least 28 days, or stability of the antibody is tested during storage at 5°C±3°C for at least 12 months).

For physical stability, the antibody in the preparation is considered to maintain its physical stability if it shows no obvious signs of aggregation, precipitation and/or denaturation by visual inspection of color and/or transparency, or by UV light scattering or by size exclusion chromatography.

For chemical stability, it can be evaluated by testing and quantifying the chemically altered form of the antibody. Chemical alterations may involve size changes (e.g., cleavage), which, for example, can be assessed by using size exclusion chromatography, SDS-PAGE, and/or matrix-assisted laser resolution ionization/time-of-flight mass spectrometry (MALDI/TOF MS). Other types of chemical alterations include charge changes (for example, changes due to deamidation), which can be assessed by ion exchange chromatography, for example.

For biological activity, if the antibody in a preparation has the biological activity for its intended use, the antibody retains its biological activity in the preparation. For example, if the biological activity of the antibody in the preparation is within about 70% to 130% (e.g., 80% to 130%) of the biological activity shown during the preparation of the preparation, it is considered that its biological activity is maintained (e.g., determined by antigen binding assay).

As used herein, the term "buffer" refers to a buffer solution that resists changes in pH through the action of its acid-base conjugated components. Examples of such buffers include acetate, succinate, gluconate, histidine, citrate, glycylglycine and other organic acid buffers. In certain preferred embodiments, the buffer suitable for use in the liquid preparations disclosed herein is selected from citric acid-citrate buffer, histidine buffer, and acetic acid-acetate buffer.

The term "histidine buffer" refers to a buffer containing histidine. The histidine in the buffer may be present alone or in the following forms: for example, histidine hydrochloride, histidine acetate, histidine phosphate or histidine sulfate. When the histidine is not present alone, the amino acid buffer (such as histidine hydrochloride buffer) can be prepared by titrating L-histidine (free base, solid) with corresponding acid (e.g., hydrochloric acid). When histidine is present in a separate form, a pH adjusting agent may be added in the subsequent preparation steps according to actual conditions to obtain a liquid preparation with a suitable pH range. In certain preferred embodiments, the histidine in the histidine buffer suitable for use in the liquid preparations disclosed herein is present alone or in the form of histidine hydrochloride or histidine acetate.

The term "citric acid-citrate buffer" includes a mixture of citric acid and citrate. In certain preferred embodiments, the citric acid-citrate buffer is a citric acid-sodium citrate buffer, that is, it contains a mixture of citric acid and sodium citrate. The citric acid-citrate buffer (e.g., citric acid-sodium citrate buffer) can be prepared by adding alkali (such as sodium hydroxide) required for salt formation to citric acid.

The term "acetic acid-acetate buffer" includes a mixture of acetic acid and acetate. In certain preferred embodiments, the acetic acid-acetate buffer is an acetic acid-sodium acetate buffer, that is, it contains a mixture of acetic acid and sodium acetate. The acetic acid-acetate buffer (e.g., acetic acid-sodium acetate buffer) can be prepared by adding alkali (such as sodium hydroxide) required for salt formation to acetic acid.

As used herein, the term "sugar" or "sugar alcohol" has the meaning commonly understood by those skilled in the art. Sugar includes the general composition (CH₂O)n and its derivatives, including monosaccharides, disaccharides, trisaccharides, polysaccharides, reducing sugars, non-reducing sugars, etc., and non-limiting examples of which herein include glucose, sucrose, trehalose, lactose, fructose, maltose, dextran, etc. Sugar alcohol can be prepared and obtained from the corresponding sugar, that is, the aldehyde group or ketone group on the sugar molecule is reduced into a hydroxyl group to obtain the sugar alcohol; a sugar alcohol has the general formula H(CHOH)nH, and non-limiting examples of which herein include erythritol, threitol, arabitol, xylitol, sorbitol, mannitol, maltitol, lactitol, etc.

The anti-PD-L1 antibody liquid preparation of the invention does not contain sugar and/or sugar alcohol, so that the preparation can also be referred to as a sugar-free antibody preparation herein. In certain preferred embodiments, the anti-PD-Ll antibody liquid preparation of the invention does not contain a sugar and/or a sugar alcohol selected from the group consisting of sucrose, trehalose, glucose, mannitol and sorbitol. It is known in the art that sugars and/or sugar alcohols (e.g., sucrose, trehalose, glucose, mannitol, and sorbitol, etc.) are usually added to antibody preparations as antibody stabilizers. In addition, sugars and/or sugar alcohols can also serve as effective osmotic pressure adjusting agents to maintain the stability of the preparation (e.g., isotonic, that is, having substantially the same osmotic pressure as human blood).

As used herein, the term "osmolality" refers to a measure of the osmotic pressure of dissolved solute particles in an aqueous solution. Solute particles include two kinds of ions and non-ionized molecules. Osmotic pressure is represented as the concentration of osmotically active particles (i.e., osmole) dissolved in 1 kg of solvent (i.e., water). Herein, the osmolality is represented in the unit of millosmoles per kilogram of water (mOsmol/kg). It is known to those skilled in the art that a desired level of osmolality can be achieved by adding one or more osmotic pressure adjusting agents, such as sugars and/or sugar alcohols.

As used herein, the term "isotonic" means that the preparation of interest has substantially the same osmotic pressure as human blood. In certain preferred embodiments, the isotonic preparation would generally have an osmotic pressure of about 260-330 mOsmol/kg. Isotonicity can be measured using, for example, a vapor pressure or a freezing-type osmometer.

As used herein, the term "mass to volume ratio" or "w/v" refers to the percentage weight (in grams) of a single component relative to the total volume (in milliliters) of the mixture containing the component. For example, 20 mg component in a total volume of 100 ml is 0.02% w/v.

As used herein, the term "about" refers to a value or composition within an acceptable error range for a specific value or composition determined by a person of ordinary skill in the art, which will depend in part on how the value or composition is measured or determined, i.e., the limitation of the measurement system. Herein, "about" means a range comprising ±5% of the given value when it is used to describe a measurable value (e.g., the concentration of a substance, time, temperature, etc.). For example, the expression "about 10mM" means 9.5-10.5 mM.

### The beneficial effects of the invention

In the process of preparing antibody preparations, it is usually necessary in the art to add sugar and/or sugar alcohol to maintain the stability of the antibody. However, the use of sugar and/or sugar alcohol has certain limitations for patients with diabetes. The inventors of the present application have, after a large number of experiments, unexpectedly discovered that the sugar-free antibody preparation of the invention can not only maintain the osmotic pressure of the antibody preparation to obtain excellent physiological compatibility, but also maintain the physical stability, chemical stability and biological activity of the preparation. It can tolerate severe conditions during storage and transportation, which is particularly advantages for long-term storage of drugs, and can expand the scope of clinical medication, which has great clinical value.

The embodiments of the invention will be described in detail below in conjunction with examples; however, those skilled in the art shall understand that the following examples are only used to illustrate the invention, but not to limit the scope of the invention. According to the following detailed description of the preferred embodiments, various purposes and advantageous aspects of the invention will become apparent to those skilled in the art.

### Sequence information

Information on partial sequences involved in the invention is provided in Table 1 below.

**Table 1: Description of the sequences**

| SEQ ID NO | Description | Sequence Information |
|---|---|---|
| 1 | Heavy chain CDR-H1 of anti-PD-L1 antibody | GFSLSNYD |
| 2 | Heavy chain CDR-H2 of anti-PD-L1 antibody | IWTGGA T |
| 3 | Heavy chain CDR-H3 of anti-PD-L1 antibody | VRDSNYRYDEPFTY |
| 4 | Light chain CDR-L1 of anti-PD-L1 antibody | QSIGTN |
| 5 | Light chain CDR-L2 of anti-PD-L1 antibody | YAS |
| 6 | Light chain CDR-L3 of anti-PD-L1 antibody | QQSNSWPYT |
| 7 | Heavy chain variable region of anti-PD-L1 antibody | QVQLQESGPGLVKPSETLSITCTVSGFSLSNYDISW IRQPPGKGLEWLGVIWTGGATNYNPALKSRLTISR DNSKNQVSLKMSSVTAADTAVYYCVRDSNYRYD EPFTYWGQGTLVTVSS |
| 8 | Light chain variable region of anti-PD-L1 antibody | EIVLTQSPDTLSVTPKEKVTLTCRASQSIGTNIHWF QQKPGQSPKLLIKYASESISGVPSRFSGSGSGTDFT LTINSVEAEDAATYYCQQSNSWPYTFGQGTKLEI K |
| 9 | Heavy chain of anti-PD-Ll antibody | QVQLQESGPGLVKPSETLSITCTVSGFSLSNYDISW IRQPPGKGLEWLGVIWTGGATNYNPALKSRLTISR DNSKNQVSLKMSSVTAADTAVYYCVRDSNYRYD EPFTYWGQGTLVTVSSASTKGPSVFPLAPSSKSTS GGTAALGCLVKDYFPEPVTVSWNSGALTSGVHTF PAVLQSSGLYSLSSVVTVPSSSLGTQTYICNVNHK PSNTKVDKKVEPKSCDKTHTCPPCPAPEAAGAPS VFLFPPKPKDTLMISRTPEVTCVVVDVSHEDPEVK FNWYVDGVEVHNAKTKPREEQYNSTYRVVSVLT VLHQDWLNGKEYKCKVSNKALPAPIEKTISKAKG QPREPQVYTLPPSRDELTKNQVSLTCLVKGFYPSD IAVEWESNGQPENNYKTTPPVLDSDGSFFLYSKLT VDKSRWQQGNVFSCSVMHEALHNHYTQKSLSLS PGK |
| 10 | Light chain of anti-PD-Ll antibody | EIVLTQSPDTLSVTPKEKVTLTCRASQSIGTNIHWF QQKPGQSPKLLIKYASESISGVPSRFSGSGSGTDFT LTINSVEAEDAATYYCQQSNSWPYTFGQGTKLEI KRTVAAPSVFIFPPSDEQLKSGTASVVCLLNNFYP REAKVQWKVDNALQSGNSQESVTEQDSKDSTYS LSSTLTLSKADYEKHKVYACEVTHQGLSSPVTKSF NRGEC |

The above-mentioned CDRs can be obtained by technical means well known to those skilled in the art, for example, by analyzing the amino acid sequence of the following heavy chain variable region or the amino acid sequence of the light chain variable region through the VBASE2 database according to IMGT definition.

### EXAMPLES

The invention will now be described with reference to the following examples which are intended to illustrate the invention but not limit the invention.

Unless otherwise specified, the experiments and methods described in the examples are basically performed according to conventional methods well known in the art and described in various references. If no specific conditions are indicated in the examples, it shall be carried out in accordance with the conventional conditions or the conditions recommended by the manufacturer. The reagents or devises used without indicating the manufacturer are all conventional products that can be purchased commercially. Those skilled in the art know that the examples illustrate the invention by way of example, and are not intended to limit the scope of protection claimed by the invention. All publications and other references mentioned herein are incorporated herein by reference in their entirety.

### Experimental materials and methods

**Table 2: Reagents involved in the examples**

| Material Name | Manufacturer |
|---|---|
| Polysorbate 80 | Nanjing Weier Chemical Co., LTD |
| Histidine hydrochloride | Shanghai Kyowa Amino Acid Co., Ltd. |
| Histidine | Shanghai Kyowa Amino Acid Co., Ltd. |
| Sodium chloride | Jiangsu Province Qinfen Pharmaceutical Co., LTD |
| Sucrose | Merck |
| Acetic acid | Hunan Er-kang Pharmaceutical Co., LTD |
| Sodium acetate | Hunan Er-kang Pharmaceutical Co., LTD |
| Citric acid | Hunan Er-kang Pharmaceutical Co., LTD |
| Sodium citrate | Hunan Er-kang Pharmaceutical Co., LTD |

The original antibody of the anti-PD-Ll humanized monoclonal antibody in the following examples is disclosed in PCT Patent Publication No. WO2017/148424, and has a heavy chain as shown in SEQ ID NO: 9 and a light chain as shown in SEQ ID NO: 10.

### Test methods

### (1) Protein concentration

The protein concentration in the sample is determined by taking about 30µl of sample and placing it in a cuvette followed by measuring the UV absorbance using a Solo-VPE variable optical path ultraviolet spectrophotometer.

### (2) Flowcam

The number of particles in the sample is determined using Flowcam GWF-8JD. About 1 ml of the sample is taken using a pipette, and the tip containing the sample is placed on the sample port to determine the number of insoluble particles ≥10 µm and ≥25 µm in the sample.

### (3) Osmotic pressure

25 µl of the sample is taken and placed in the sample tube, which is then placed under the probe of 819M freezing point osmotic pressure meter (Loser) to measure the osmotic pressure of the sample.

### (4) Size exclusion chromatography (SEC-HPLC)

The processed sample is injected and eluted in the mobile phase for about 20 mins on Waters e2695 HPLC using TSKgel G3000SWXL column with the column temperature kept at room temperature. The distribution of large and small molecules in the sample is determined by size exclusion chromatography. The sample curve is obtained based on the UV absorption at 280 nm, and the relative percentage peak area of the total area is reported as the result.

### (5) Ion exchange chromatography (IEC-HPLC)

The processed sample is injected and eluted in the mobile phase on Waters e2695 HPLC using ProPac WCX-10 column with the column temperature kept at 30°C. The distribution of different charged ion components in the sample is determined by ion exchange chromatography. The sample curve is obtained based on the UV absorption at 280 nm, and the relative percentage peak area of the total area is reported as the result.

### (6) Capillary electrophoresis (CE-SDS)

After the sample is processed, it is quantitatively determined for the purity of the recombinant monoclonal antibody product based on the molecular weight under non-reduced (NR) conditions, using sodium dodecyl sulfate capillary electrophoresis (CE-SDS) ultraviolet test method.

### (7) Relative binding activity (%)

The EC50 (half effective concentration) of the sample is tested by establishing an enzyme-linked immunoassay (ELISA). By comparing the EC50 of the sample and the reference, the relative binding activity of the sample is calculated. Since the detection deviation of this method is ±20%, the standard is initially determined to be 70-130%.

### Example 1

Formulation 1: anti-PD-Ll humanized monoclonal antibody at 20 mg/ml, 100 mM sodium chloride, 20 mM citric acid-sodium citrate, pH 5.2.

Formulation 2: anti-PD-Ll humanized monoclonal antibody at 20 mg/ml, 100 mM sodium chloride, 20 mM citric acid-sodium citrate, pH 5.6.

Formulation 3: anti-PD-Ll humanized monoclonal antibody at 20 mg/ml, 100 mM sodium chloride, 20 mM citric acid-sodium citrate, pH 6.0.

Formulation 4: anti-PD-Ll humanized monoclonal antibody at 20 mg/ml, 100 mM sodium chloride, 20 mM citric acid-sodium citrate, pH 6.4.

Formulation 5: anti-PD-Ll humanized monoclonal antibody at 20 mg/ml, 100 mM sodium chloride, 20 mM citric acid-sodium citrate, pH 6.8.

The preparation methods of the antibody preparations of Formulations 1-5 are as follows:
1. Buffer preparation: 100 mM sodium chloride and 20 mM citric acid were added to the water for injection, and adjusted to the desired pH value in the above formulation with sodium hydroxide.
2. The antibody stock solution (Sichuan Kelun Botai Bio-Pharmaceutical Co., Ltd., Batch No. 1) was dialyzed by ultrafiltration and liquid exchange into the above buffer, and the antibody concentration was adjusted to 20 mg/ml.
3. The prepared sample was sterilized by filtration through a 0.22 µm pore size filter under laminar flow, and filled into a vial which was then sealed with a stopper and a cap to obtain the sample.

The effect of the pH value of the preparation on the stability of the monoclonal antibody was investigated by the DSF method. The results showed that the Tm values of the samples with different pH values were all greater than 65°C, indicating that the pH value in the range of pH 5.6-6.8 does not have a significant effect on the thermal stability of the protein (Table 3). The sample was placed at 40°C for 3 days for accelerated experiment. The SEC-HPLC results showed that the monomer was greater than 99.0% with no significant decrease, no oligomer was detected, and the high polymer was less than 0.20% with no significant increase. The stability of the samples was good (Table 4).

**Table 3: Tm values of samples with different pH values**

| Samples | Formulation 1 | Formulation 2 | Formulation 3 | Formulation 4 | Formulation 5 |
|---|---|---|---|---|---|
| DSF Tm(°C) | 65.31 | 66.25 | 66.25 | 66.33 | 66.40 |

**Table 4: Accelerated experiment results of samples with different pH values**

| Samples | Storage conditions and time | SEC-HPLC | | |
|---|---|---|---|---|
| | | Monomer (%) | Oligomer (%) | Polymer (%) |
| Formulation 1 | 0 day | 99.86 | 0 | 0.14 |
| | 40°C 3 days | 99.88 | 0 | 0.12 |
| Formulation 2 | 0 days | 99.87 | 0 | 0.13 |
| | 40°C 3 days | 99.87 | 0 | 0.13 |
| Formulation 3 | 0 day | 99.86 | 0 | 0.14 |
| | 40°C 3 days | 99.86 | 0 | 0.14 |
| Formulation 4 | 0 day | 99.84 | 0 | 0.16 |
| | 40°C 3 days | 99.84 | 0 | 0.16 |
| Formulation 5 | 0 day | 99.85 | 0 | 0.15 |
| | 40°C 3 days | 99.85 | 0 | 0.15 |

### Example 2

Formulation 6: anti-PD-Ll humanized monoclonal antibody at 20 mg/ml, 140 mM sodium chloride, 20 mM histidine-histidine hydrochloride, polysorbate 80 at a mass-to-volume ratio of 0.02%, pH 5.8.

100 ml of the antibody preparation of Formulation 6 was prepared with the preparation method as follows:
1. Buffer preparation: 140 mM sodium chloride and 20 mM histidine were added to the water for injection, and adjusted to pH 5.8 with hydrochloric acid.
2. The antibody stock solution (Batch No. 1) was dialyzed by ultrafiltration and liquid exchange into the above buffer, the antibody concentration was adjusted to 20 mg/ml, and an appropriate amount of polysorbate 80 stock solution was added.
3. The prepared sample was sterilized and filtered through a 0.22 µm pore size filter under laminar flow, and filled into a vial which was then sealed with a stopper and a cap to obtain the sample.

The stability of the sample of Formulation 6 at 40°C for 28 days was investigated, and the testing results are shown in Tables 5-9, respectively.

### Example 3

Formulation 7: anti-PD-Ll humanized monoclonal antibody at 20 mg/ml, 140 mM sodium chloride, 20 mM histidine-histidine hydrochloride, polysorbate 80 at a mass-to-volume ratio of 0.02%, pH 5.0.

The pH of the buffer was adjusted to 5.0, and the rest of the preparation method is the same as in Example 2.

The stability of the sample of Formulation 7 at 40°C for 28 days was investigated, and the testing results are shown in Tables 5-9, respectively.

### Example 4

Formulation 8: anti-PD-Ll humanized monoclonal antibody at 20 mg/ml, 140 mM sodium chloride, 20 mM histidine-histidine hydrochloride, polysorbate 80 at a mass-to-volume ratio of 0.02%, pH 7.0.

The pH of the buffer was adjusted to 7.0, and the rest of the preparation method is the same as in Example 2.

The stability of the sample of Formulation 8 at 40°C for 28 days was investigated, and the testing results are shown in Tables 5-9, respectively.

### Example 5

Formulation 9: anti-PD-Ll humanized monoclonal antibody at 20 mg/ml, 140 mM sodium chloride, 20 mM histidine-histidine acetate, polysorbate 80 at a mass-to-volume ratio of 0.02%, pH 5.8.

140 mM sodium chloride and 20 mM histidine were added to the water for injection, and the pH was adjusted to 5.8 with acetic acid. The rest of the preparation method is the same as in Example 2.

The stability of the sample of Formulation 9 at 40°C for 28 days was investigated, and the testing results are shown in Tables 5-9, respectively.

### Example 6

Formulation 10: anti-PD-Ll humanized monoclonal antibody at 20 mg/ml, 140 mM sodium chloride, 20 mM acetic acid-sodium acetate, polysorbate 80 at a mass-to-volume ratio of 0.02%, pH 5.8.

140 mM sodium chloride and 20 mM sodium acetate were added to the water for injection, and the pH was adjusted to 5.8 with acetic acid. The rest of the preparation method is the same as in Example 2.

The stability of the sample of Formulation 10 at 40°C for 28 days was investigated, and the testing results are shown in Tables 5-9, respectively.

### Example 7

Formulation 11: anti-PDL-1 humanized monoclonal antibody at 20 mg/ml, 140 mM sodium chloride, 20 mM citric acid-sodium citrate, polysorbate 80 at a mass-to-volume ratio of 0.02%, pH 5.8.

140 mM sodium chloride and 20 mM sodium citrate were added to the water for injection, and the pH was adjusted to 5.8 with citric acid. The rest of the preparation method is the same as in Example 2.

The stability of the sample of Formulation 11 at 40°C for 28 days was investigated, and the testing results are shown in Tables 5-9, respectively.

### Example 8

Formulation 12: anti-PD-Ll humanized monoclonal antibody at 20 mg/ml, 140 mM sodium chloride, 20 mM histidine-histidine hydrochloride, polysorbate 20 at a mass-to-volume ratio of 0.02%, pH 5.8.

In Formulation 8, polysorbate 80 was replaced with polysorbate 20, and the rest of the preparation method is the same as in Example 2.

The stability of the sample of Formulation 12 at 40°C for 28 days was investigated, and part of the testing results are shown in Tables 7-9, respectively.

### Example 9

The stability of the samples of the above-mentioned Examples 2-8 placed under the condition of 40°C for 28 days was tested.

### 9.1 Test of osmotic pressure

The testing results for osmotic pressure of Examples 2-7 are shown in the following table. The results showed that, upon placed under the condition of 40°C for 28 days, the osmotic pressure of the samples of Examples 2-7 can be stable in the range of 280-320 mOsmol/kg with good stability.

**Table 5: Testing results of osmotic pressure**

| Osmotic Pressure (mOsmol/kg) | 40°C 0 day | 40°C 14 days | 40°C 28 days |
|---|---|---|---|
| Example 2 | 297 | 293 | 295 |
| Example 3 | 310 | 306 | 304 |
| Example 4 | 294 | 301 | 292 |
| Example 5 | 309 | 302 | 289 |
| Example 6 | 307 | 305 | 306 |
| Example 7 | 318 | 318 | 315 |

### 9.2 Test of insoluble particles

The insoluble particles of the samples of Examples 2-7 were tested by Flowcam, and the results are shown in the following table. The results showed that the number of particles ≥ 10 µm in the samples of each formulation was less than 1000, and the number of particles ≥ 25 µm was less than 200, indicating that there was no obvious aggregation in Formulations 6-11 after being placed at 40°C for 28 days, which meets the requirements for medicinal use.

**Table 6: Testing results of Flowcam**

| Counts | 10 µm | | 25 µm | |
|---|---|---|---|---|
| | 40°C 0 day | 40°C 28 days | 40°C 0 day | 40°C 28 days |
| Example 2 | 201 | 306 | 11 | 49 |
| Example 3 | 106 | 198 | 18 | 33 |
| Example 4 | 166 | 639 | 25 | 119 |
| Example 5 | 92 | 231 | 18 | 46 |
| Example 6 | 155 | 853 | 7 | 135 |
| Example 7 | 519 | 221 | 71 | 56 |

### 9.3 Test by size exclusion chromatography (SEC-HPLC)

The monomer content in the samples of Examples 2-8 was tested by size exclusion chromatography, and the results are shown in the following table. The results showed that the monomer content of the samples of Formulations 6-12 were all greater than 95%, which remained stable after being placed at 40°C for 28 days.

**Table 7: Testing results of SEC-HPLC**

| Content (%) | Monomer | | |
|---|---|---|---|
| | 40°C 0 day | 40°C 14 days | 40°C 28 days |
| Example 2 | 99.3 | 98.9 | 97.1 |
| Example 3 | 99.4 | 97.1 | 95.6 |
| Example 4 | 99.4 | 98.9 | 97.3 |
| Example 5 | 99.4 | 99.0 | 97.4 |
| Example 6 | 99.3 | 98.9 | 97.2 |
| Example 7 | 99.4 | 96.9 | 96.2 |
| Example 8 | 99.4 | 99.1 | 97.2 |

### 9.4 Test by ion exchange chromatography (IEC-HPLC)

The main peak content of the samples of Examples 2-8 was tested by ion exchange chromatography, and the results are shown in the following table. The results showed that the main peak content of Formulations 6-12 which were placed at 40°C for 28 days remained stable, which meets the quality requirements.

**Table 8: Testing results of IEC-HPLC**

| Content (%) | Main Peak | | |
|---|---|---|---|
| | 40°C 0 day | 40°C 14 days | 40°C 28 days |
| Example 2 | 65.3 | 60.3 | 55.6 |
| Example 3 | 65.2 | 53.5 | 48.6 |
| Example 4 | 65.3 | 56.9 | 49.0 |
| Example 5 | 65.3 | 59.2 | 54.0 |
| Example 6 | 65.3 | 59.0 | 54.1 |
| Example 7 | 65.3 | 56.5 | 49.0 |
| Example 8 | 65.2 | 61.4 | 56.3 |

### 9.5 Test by Capillary electrophoresis (CE-SDS)

The purity of main peak of the samples of Examples 2-8 was tested by capillary electrophoresis, and the results are shown in the following table. The results showed that after being placed at 40°C for 28 days, the purity of main peak of the samples of Formulations 6-12 were high and the content remained stable, which meets the quality requirements.

**Table 9: Testing results of non-reducing CE-SDS**

| Main Peak Purity (%) | 40°C 0 day | 40°C 14 days | 40°C 28 days |
|---|---|---|---|
| Example 2 | 96.3 | 95.9 | 95.0 |
| Example 3 | 97.0 | 95.8 | 89.2 |
| Example 4 | 97.0 | 95.3 | 94.0 |
| Example 5 | 96.8 | 95.9 | 93.4 |
| Example 6 | 97.0 | 95.7 | 93.1 |
| Example 7 | 97.0 | 95.1 | 91.3 |
| Example 8 | 97.2 | 95.0 | 93.4 |

### Example 10

Formulation 6: anti-PD-Ll humanized monoclonal antibody at 20 mg/ml, 140 mM sodium chloride, 20 mM histidine-histidine hydrochloride, polysorbate 80 at a mass-to-volume ratio of 0.02%, pH 5.8.

The antibody preparation of Formulation 6 was enlarged to 10 L with the preparation method as follows:
1. Preparation of ultrafiltration buffer: sodium chloride and histidine were added to the water for injection, and the pH value was adjusted to 5.8 with hydrochloric acid to prepare a solution containing 140 mM sodium chloride and 20 mM histidine-histidine hydrochloride.
2. The antibody stock solution (Batch No. 2) was ultrafiltered and liquid exchanged using Millipore ultrafiltration membrane packs by selecting an appropriate number of membrane packs according to the load, using the Thermo Pellicon ultrafiltration system. The protein was dialyzed into the above buffer, the antibody concentration was adjusted to 20 mg/ml, and an appropriate amount of polysorbate 80 was added.
3. The prepared sample was sterilized and filtered under laminar flow conditions. The filtration was performed using 0.22 µm filter of Millipak series (Millipore). If necessary, a pre-filtration membrane Opticap filter can be connected in series before the sterilization filter to increase the filter capacity. In a sterile environment (ultra-clean bench), the sterilization filter was connected to the peristaltic pump with a silica gel tube, followed by a 0.22 µm pore size filter for sterilization and filtration. The filtered sample solutions were collected and filled into vials separately, which were then sealed with stoppers and caps to obtain the samples.

A long-term study of 12-month stability at 5°C±3°C was carried out. The items investigated were protein concentration, SEC-HPLC, CE-SDS, and IEC-HPLC, and the results are shown in Table 10. The results showed that the anti-PD-Ll humanized monoclonal antibody liquid preparation of this formulation can be scaled up, and can still achieve the same quality as small-scale preparations after scale-up production. The antibody concentration was stable, the purity remained high, and the stability was good. It can be stored stably for at least 12 months under the temperature of 5°C±3°C.

**Table 10: Testing data for long-term stability of Formulation 6**

| | | | | |
|---|---|---|---|---|
| Storage Time at 5°C±3°C | | 0 month | 6 months | 12 months |
| Protein Concentration (mg/ml) | | 20.6 | 21.6 | 20.7 |
| pH value | | 5.8 | 5.6 | 5.6 |
| SEC-HPLC | Monomer (%) | 99.27 | 98.78 | 98.70 |
| CE-SDS | Non-Reducing (%) | 95.35 | 95.64 | 94.39 |
| IEC-HPLC | Main Peak (%) | 71.91 | 67.46 | 65.93 |

In addition, a 48-hour freeze-thaw stability study was conducted for Formulation 6 at -20°C. The items investigated were OD405, SEC-HPLC and IEC-HPLC. The results are shown in Table 11. The results showed that the anti-PD-Ll humanized monoclonal antibody liquid preparation of this formulation has good stability during 4 cycles of freezing and thawing, and can tolerate the severe conditions of the preparation during storage and transportation.

**Table 11: Testing data for freeze-thaw stability of Formulation 6**

| Freeze-Thaw Cycle | OD405 | SEC-HPLC | IEC-HPLC |
|---|---|---|---|
| | | Monomer (%) | Main Peak (%) |
| 0 | 0.013 | 99.53 | 69.30 |
| 1 | 0.017 | 99.55 | 69.42 |
| 2 | 0.015 | 99.49 | 67.62 |
| 4 | 0.013 | 99.50 | 67.68 |

Another batch of the antibody stock solution (Batch No. 3) of the preparations with the same formulation was prepared using the same method as described above in this example. The long-term stability study at 5±3°C and the accelerated stability study at 25±2°C were carried out for Formulation 6. The items investigated were appearance, protein concentration, pH, osmotic pressure, insoluble particles, SEC-HPLC, CE-SDS, IEC-HPLC, and binding activity. The results are as follows:

**Table 12: Testing data for long-term stability study at 5±3°C of Formulation 6**

| | | | | | | |
|---|---|---|---|---|---|---|
| Investigation Time | At the beginning | 6 months | 12 months | 24 months | 36 months | |
| Appearance | Colorless and clear liquid | Colorless and clear liquid | Colorless and clear liquid | Colorless and clear liquid | Colorless and clear liquid | |
| Protein Concentration (mg/ml) | 20.6 | 20.7 | 20.3 | 20.8 | 20.7 | |
| Investigation Time | | At the beginning | 6 months | 12 months | 24 months | 36 months |
| pH | | 5.9 | 6.0 | 6.0 | 6.0 | 6.0 |
| Osmolality (mOsmol/kg) | | 291 | 296 | 298 | 299 | 292 |
| Insoluble Particles (number) | ≥ 10 µM | 873 | 76 | 184 | 68 | 80 |
| | ≥ 25 µM | 1 | 16 | 10 | 2 | 2 |
| CE-SDS Reduced (%) | | 99.1 | 99.2 | 98.6 | 98.9 | 99.2 |
| CE-SDS Non-Reduced (%) | | 96.7 | 96.7 | 96.7 | 96.9 | 96.9 |
| SEC-HPLC Main Peak (%) | | 99.7 | 99.5 | 99.4 | 99.3 | 99.3 |
| IEC-HPLC Main Peak (%) | | 71.9 | 71.3 | 70.9 | 70.2 | 69.6 |
| Relative Binding Activity (%) | | 100 | 90 | 112 | 103 | 83 |

Formulation 6 was placed at 5°C for a long time of 36 months. The appearance was clear and transparent, and the protein content, pH, osmolality, CE-SDS reduced and non-reduced, SEC-HPLC, IEC-HPLC and relative binding activity did not change significantly, showing good stability.

**Table 13: Testing data for accelerated stability study at 25°C±2°C of Formulation 6**

| | | | | | |
|---|---|---|---|---|---|
| Investigation Time | At the beginning | 1 months | 3 months | 6 months | |
| Appearance | Colorless and clear liquid | Colorless and clear liquid | Colorless and clear liquid | Colorless and clear liquid | |
| Protein Concentration (mg/ml) | 20.6 | 20.8 | 19.8 | 21.2 | |
| pH | | 5.9 | 5.9 | 6.0 | 6.0 |
| Osmolality (mOsmol/kg) | | 291 | 287 | 282 | 292 |
| Insoluble Particles (number) | ≥ 10 µm | 873 | 14 | 120 | 113 |
| | ≥ 25 µm | 1 | 1 | 5 | 6 |
| CE-SDS Reduced (%) | | 99.1 | 99.2 | 99.2 | 97.7 |
| CE-SDS Non-Reduced (%) | | 96.7 | 96.9 | 96.0 | 94.8 |
| SEC-HPLC Main Peak (%) | | 99.7 | 99.5 | 99.3 | 97.6 |
| IEC-HPLC Main Peak (%) | | 71.9 | 69.9 | 66.5 | 63.0 |
| Relative Binding Activity (%) | | 100 | 102 | 82 | 86 |

Formulation 6 was placed under accelerated conditions at 25±2°C for 6 months. The appearance was clear and transparent. There was no significant change in protein content, pH, osmotic pressure and relative binding activity. The purity for CE-SDS reduced and non-reduced, SEC-HPLC and IEC-HPLC was slightly decreased, and the stability of the protein was good, which meets the quality requirements.

### Example 11

Formulation 16: anti-PD-Ll humanized monoclonal antibody at 60 mg/ml, 10 mM histidine-histidine hydrochloride, 140 mM sodium chloride, polysorbate 80 at a mass-to-volume ratio of 0.02%, pH 5.7. The antibody concentration in the buffer was 60 mg/ml, and the rest of the preparation method was the same as in Example 2.

The testing results of the stability at 40°C are shown in Table 14. The appearance of the sample of Formulation 16 was clear and transparent liquid with slight opalescence; and over time at 40°C, the OD405 was stable, indicating that the formulation is stable.

**Table 14: Testing results for stability of Formulation 16**

| Investigation Condition | 40°C 0 day | 40°C 3 days | 40°C 7 days | 40°C 14 days |
|---|---|---|---|---|
| Appearance | Clear and transparent liquid with slight opalescence | | | |
| OD405 | 0.040 | 0.047 | 0.043 | 0.048 |

### Example 12

Formulation 17: anti-PD-Ll humanized monoclonal antibody at 60 mg/ml, 20 mM histidine-histidine hydrochloride, 140 mM sodium chloride, 0.02% w/v polysorbate 80, pH5.8; the preparation method was the same as in Example 2.

Formulation 18: anti-PD-Ll humanized monoclonal antibody at 90 mg/ml, 20 mM histidine-histidine hydrochloride, 140 mM sodium chloride, 0.02% w/v polysorbate 80, pH5.8; the preparation method was the same as in Example 2.

Formulation 19: anti-PD-Ll humanized monoclonal antibody at 120 mg/ml, 20 mM histidine-histidine hydrochloride, 140 mM sodium chloride, 0.02% w/v polysorbate 80, pH5.8; the preparation method was the same as in Example 2.

The viscosity of Formulation 6, Formulation 17, Formulation 18 and Formulation 19 and the change of viscosity under different temperature conditions were investigated respectively. The results are shown in Table 15. The viscosity of Formulation 6 was low under the low concentration condition and as the concentration increased, the viscosity of Formulations 17, 18 and 19 gradually increased. Formulation 6 and Formulation 17 were placed at 5°C for 6 months, and the viscosity did not change significantly compared with that at the beginning; Formulation 18 and Formulation 19 were placed at 5°C and 25°C for 3 months, with no significant change in the viscosity compared to that at the beginning. High-concentration preparations usually have high viscosity, and the viscosity of the preparations of the present application was controllable and stable at high concentrations, which meets the quality requirements.

**Table 15: The viscosity and viscosity values of Formulations 6 and 17-19 under different temperature conditions**

| Samples | Protein Concentration (mg/ml) | Viscosity/cp | | | | |
|---|---|---|---|---|---|---|
| | | At the beginning | 25°C 3 months | 5°C 3 months | 25°C 6 months | 5°C 6 months |
| Formulation 6 | 20 | 1.28 | NT | NT | NT | 1.12 |
| Formulation 17 | 60 | 1.79 | NT | NT | 1.77 | 1.74 |
| Formulation 18 | 90 | 3.11 | 3.24 | 3.22 | NT | NT |
| Formulation 19 | 120 | 6.50 | 6.87 | 6.89 | NT | NT |

| | | | | | | |
|---|---|---|---|---|---|---|
| NT stands for not tested. | | | | | | |

### Example 13

The stability of Formulation 18 and Formulation 19 under shaking, 40°C, 25°C and 5°C were investigated respectively. The results are shown in Table 16. Under the condition of continuous shaking at 1000 rpm for 2h, the items tested for Formulation 18 and Formulation 19 did not change significantly, and the stability was good. When placed under the condition of 5°C for 2 months, Formulation 19 had the physical and chemical items tested remained substantially unchanged with good stability, indicating that the finished preparation of Formulation 19 can be placed for at least 2 months under the condition of 5±3°C. Placed under the accelerated condition at 25°C for 2 months, Formulation 18 and Formulation 19 had the main IEC peaks slightly reduced with the rest of the items tested substantially unchanged, indicating that the finished preparations of Formulation 18 and Formulation 19 can be placed at least 2 months under the condition of 25±2°C. When placed at 40°C for 13 days, Formulation 18 and Formulation 19 had a slight decrease in the IEC main peaks and CE-SDS non-reduced main peaks with the rest of the items tested not significantly changed, which meets the quality requirements, indicating that the finished preparations of Formulation 18 and Formulation 19 can be placed for at least 13 days under the condition of 40°C. The preparations of the application overcome the problems of stability, viscosity, osmotic pressure and turbidity, which is beneficial to long-term storage of drugs and clinical medication.

**Table 16: Testing results for stability of Formulation 18 and Formulation 19 under the condition of shaking at 40°C, 25°C and 5°C.**

| Formulation | Investigation Time | At the beginning | Shaking | 40°C | | 25°C | | 5°C |
|---|---|---|---|---|---|---|---|---|
| | | | 2h | 7 days | 13 days | 1 month | 2 months | 2 months |
| Formulation 18 | Appearance | Clear and transparent liquid with opalescence | | | | | | |
| | OD405 | 0.136 | 0.138 | 0.138 | 0.130 | 0.135 | 0.146 | NT |
| | CE-SDS Reduced (%) | 97.4 | 97.5 | 96.5 | 96.5 | 96.6 | 96.5 | NT |
| | CE-SDS Non-Reduced (%) | 95.7 | 95.4 | 95.3 | 93.9 | 95.4 | 94.9 | NT |
| | SEC-HPLC Main Peak (%) | 99.1 | 99.1 | 98.8 | 98.6 | 99.0 | 98.8 | NT |
| | IEC-HPLC Main Peak (%) | 64.2 | 64.0 | 60.9 | 58.2 | 63.4 | 62.2 | NT |
| Formulation 19 | Appearance | Clear and transparent liquid with opalescence | | | | | | |
| | OD405 | 0.168 | 0.161 | 0.168 | 0.166 | 0.166 | 0.176 | 0.176 |
| | CE-SDS Reduced (%) | 97.5 | 97.3 | 96.9 | 96.8 | 96.6 | 96.5 | 97.0 |
| | CE-SDS Non-Reduced (%) | 95.8 | 95.8 | 95.3 | 94.3 | 95.4 | 94.8 | 95.6 |
| | SEC-HPLC Main Peak (%) | 99.2 | 99.1 | 98.5 | 98.5 | 99.0 | 98.7 | 99.0 |
| | IEC-HPLC Main Peak (%) | 64.3 | 64.0 | 61.1 | 58.3 | 63.5 | 62.3 | 65.0 |

| | | | | | | | | |
|---|---|---|---|---|---|---|---|---|
| NT stands for not tested. | | | | | | | | |

### Comparative example 1

Formulation 13: anti-PD-Ll humanized monoclonal antibody at 20 mg/ml, 140 mM sucrose, 20 mM histidine-histidine hydrochloride, polysorbate 80 at a mass-to-volume ratio of 0.02%, pH 5.8. The 140 mM sodium chloride in the buffer was replaced with 140 mM sucrose, and the rest of the preparation method is the same as in Example 2.

Formulation 14: anti-PD-Ll humanized monoclonal antibody at 20 mg/ml, 105 mM sucrose, 35 mM sodium chloride, 20 mM histidine-histidine hydrochloride, polysorbate 80 at a mass-to-volume ratio of 0.02%, pH 5.8. The 140 mM sodium chloride in the buffer was replaced with 105 mM sucrose and 35 mM sodium chloride, and the rest of the preparation method is the same as in Example 2.

Formulation 15: anti-PD-Ll humanized monoclonal antibody at 20 mg/ml, 70 mM sucrose, 70 mM sodium chloride, 20 mM histidine-histidine hydrochloride, polysorbate 80 at a mass-to-volume ratio of 0.02%, pH 5.8. The 140 mM sodium chloride in the buffer was replaced with 70 mM sodium chloride and 70 mM sucrose, and the rest of the preparation method is the same as in Example 2.

After detection, the osmotic pressures of Formulations 13-15 were 199 mOsmol/kg, 232 mOsmol/kg and 258 mOsmol/kg, respectively, which were all lower than that of normal human plasma. The antibody preparation is a hypotonic solution, for which intravenous administration has the risk of hemolysis, which does not meet the needs of clinical medication.

A summary of all the Formulations and their respective ingredients involved in the examples of the invention is shown in Table 17.

**Table 17 Numbering and respective ingredients of each of the formulations**

| **Formula tion No.** | **Antibody (mg/ml)** | **NaCl (mM)** | **Sucrose (mM)** | **Buffer pair and its concentration (mM)** | **pH** | **Surfactant** |
|---|---|---|---|---|---|---|
| 1 | 20 | 100 | | 20 mM Citric Acid-Sodium Citrate | 5.2 | |
| 2 | 20 | 100 | | 20 mM Citric Acid-Sodium Citrate | 5.6 | |
| 3 | 20 | 100 | | 20 mM Citric Acid-Sodium Citrate | 6.0 | |
| 4 | 20 | 100 | | 20 mM Citric Acid-Sodium Citrate | 6.4 | |
| 5 | 20 | 100 | | 20 mM Citric Acid-Sodium Citrate | 6.8 | |
| 6 | 20 | 140 | | 20 mM Histidine-Histidine Hydrochloride | 5.8 | 0.02%(w/v) Polysorbate 80 |
| 7 | 20 | 140 | | 20 mM Histidine-Histidine Hydrochloride | 5.0 | 0.02%(w/v) Polysorbate 80 |
| 8 | 20 | 140 | | 20 mM Histidine-Histidine Hydrochloride | 7.0 | 0.02%(w/v) Polysorbate 80 |
| 9 | 20 | 140 | | 20 mM Histidine-Histidine Acetate | 5.8 | 0.02%(w/v) Polysorbate 80 |
| 10 | 20 | 140 | | 20 mM Acetic acid-sodium acetate | 5.8 | 0.02%(w/v) Polysorbate 80 |
| 11 | 20 | 140 | | 20 mM Citric Acid-Sodium Citrate | 5.8 | 0.02%(w/v) Polysorbate 80 |
| 12 | 20 | 140 | | 20 mM Histidine-Histidine Hydrochloride | 5.8 | 0.02%(w/v) Polysorbate 20 |
| 13 | 20 | | 140 | 20 mM Histidine-Histidine Hydrochloride | 5.8 | 0.02%(w/v) Polysorbate 80 |
| 14 | 20 | 35 | 105 | 20 mM Histidine-Histidine Hydrochloride | 5.8 | 0.02%(w/v) Polysorbate 80 |
| 15 | 20 | 70 | 70 | 20 mM Histidine-Histidine Hydrochloride | 5.8 | 0.02%(w/v) Polysorbate 80 |
| 16 | 60 | 140 | | 10 mM Histidine-Histidine Hydrochloride | 5.7 | 0.02%(w/v) Polysorbate 80 |
| 17 | 60 | 140 | | 20 mM Histidine-Histidine Hydrochloride | 5.8 | 0.02%(w/v) Polysorbate 80 |
| 18 | 90 | 140 | | 20 mM Histidine-Histidine Hydrochloride | 5.8 | 0.02%(w/v) Polysorbate 80 |
| 19 | 120 | 140 | | 20 mM Histidine-Histidine Hydrochloride | 5.8 | 0.02%(w/v) Polysorbate 80 |

First, the experiments of Tm value assay and the stability assay under the condition of 40°C were performed on the antibody preparations of Formulations 1-5. The results showed that the antibody preparations of Formulations 1-5 of the invention have good stability, which also indicates that when the pH value is in the range of 5.6-6.8, the PD-L1 antibody of the invention has a good stability.

Subsequently, the antibody preparations of Formulations 6-12 were subjected to osmotic pressure monitoring, insoluble particle monitoring, SEC-HPLC detection, IEC-HPLC detection, and CE-SDS detection under the condition of 40°C for 28 days. The results showed that the antibody preparations of Formulations 6-12 of the invention have good stability with no obvious aggregation, which all meet the requirements of medicinal use. In addition, the results showed that the stability of the antibody of the invention is best when the pH is 5.8, and when the buffer pair in the formulation is histidine-histidine hydrochloride, the stability is relatively better than other buffer pairs.

Therefore, the present inventors conducted further tests and analysis on the antibody preparation of Formulation 6 which is with relative better effects. Specifically, the long-term stability for 12 months at 5°C±3°C and the long-term stability of storage at 5°C for 36 months were analyzed, and the protein concentration, SEC-HPLC, CE-SDS, IEC-HPLC, binding activity, etc. were tested respectively; further, its freeze-thaw stability at -20°C for 48 hours was analyzed, and OD405, SEC-HPLC and IEC-HPLC were tested respectively. The results showed that the antibody preparation of Formulation 6 can be stored stably for at least 12 months at 5°C±3°C, and can still achieve the same product quality as small-scale preparations after scale-up production. After repeated freezing and thawing for 4 cycles, the stability was good, and it can tolerate the severe conditions of the preparation during storage and transportation. In addition, after long-term storage at 5°C for 36 months, the appearance was clear and transparent with no significant changes in protein content, pH, osmolality, CE-SDS reduced and non-reduced, SEC-HPLC, IEC-HPLC and relative binding activity, and the stability is good.

Furthermore, the inventors tried antibody preparations with high antibody concentrations-Formulations 16-19, and tested the stability of the antibody preparations when the PD-L1 antibody concentration was 60 mg/ml, 90 mg/ml and 120 mg/ml, respectively. The results showed that under the condition of 40°C, the appearance of the antibody preparation of Formulation 16 was clear and transparent liquid with slight opalescence, and the OD405 was stable; under different temperature conditions, the viscosity of the antibody preparations of Formulations 17-19 did not change significantly over time; the antibody preparations of Formulations 18 and 19 had good stability after continuous shaking at 1000 rpm for 2 hours, and can be stored at 5°C and 25°C for 2 months and at 40°C for 13 days. Therefore, in the invention, the antibody preparations with a high concentration of antibody can overcome the problems of stability, viscosity, osmotic pressure and turbidity, which is beneficial for long-term storage of drugs and clinical medication.

In addition, the inventors tested the concentration of NaCl in the antibody preparation-Formulations 13-15 (comparative examples). The results showed that when the NaCl concentration was 0 mM, 35 mM and 70 mM, the osmotic pressure was 199 mOsmol/kg, 232 mOsmol/kg and 258 mOsmol/kg, respectively, which were all lower than that of normal human plasma. It has the risk of hemolysis when administrated intravenously, which does not meet the needs of clinical medication.

Although the specific embodiments of the invention have been described in detail, those skilled in the art will understand that according to all the teachings that have been published, various modifications and changes can be made to the details, and these alterations are within the protection scope of the invention. All of the invention is given by the appended claims and any equivalents thereof.

## Claims

1. A liquid preparation comprising:
(1) an anti-PD-Ll antibody at a concentration of about 10 mg/ml to about 150 mg/ml; wherein the anti-PD-Ll antibody comprises heavy chain CDRs as shown in SEQ ID NOs: 1-3 respectively, and light chain CDRs as shown in SEQ ID NOs: 4-6, respectively;
(2) a buffer at a concentration of about 5 mM to about 40 mM;
(3) sodium chloride at a concentration of about 80 mM to about 160 mM;
wherein the liquid preparation has a pH of about 5.0 to about 7.0, and the liquid preparation has no added sugar and/or sugar alcohol;
and the liquid preparation has an osmotic pressure comparable to that of human blood.

2. The liquid preparation of claim 1, wherein the anti-PD-Ll antibody has a heavy chain variable region as shown in SEQ ID NO: 7 and a light chain variable region as shown in SEQ ID NO: 8;
preferably, the anti-PD-Ll antibody has a heavy chain as shown in SEQ ID NO: 9 and a light chain as shown in SEQ ID NO: 10.

3. The liquid preparation of claim 1 or 2, wherein the concentration of the anti-PD-Ll antibody is about 15 mg/ml to about 25 mg/ml, about 50 mg/ml to about 70 mg/ml, or about 80 mg/ml to about 150mg/ml;
preferably, the concentration of the anti-PD-Ll antibody is about 20 mg/ml, about 60 mg/ml, about 90 mg/ml or about 120 mg/ml.

4. The liquid preparation of any one of claims 1-3, wherein the liquid preparation does not contain a sugar and/or a sugar alcohol selected from the group consisting of sucrose, glucose, trehalose, mannitol and sorbitol.

5. The liquid preparation of any one of claims 1-4, wherein the concentration of the sodium chloride is about 100 mM to about 160 mM, such as about 110 mM, about 120 mM, about 130 mM, about 140 mM, about 150 mM, or 155 mM;
preferably, the concentration of the sodium chloride is about 140 mM.

6. The liquid preparation of any one of claims 1-5, wherein the buffer is selected from: citric acid-citrate buffer, histidine buffer or acetic acid-acetate buffer; the histidine is present alone or in the form of histidine-hydrochloride or histidine-acetate in the histidine buffer.

7. The liquid preparation of any one of claims 1-6, wherein the concentration of the buffer is about 10 mM to about 20 mM.

8. The liquid preparation of any one of claims 1-7, wherein the liquid preparation has a pH of about 5.2 to about 7.0;
preferably, the liquid preparation has a pH of about 5.6 to about 6.8;
preferably, the liquid preparation has a pH of about 5.6 to about 6.0; preferably, the liquid preparation has a pH of 5.8±0.1.

9. The liquid preparation of any one of claims 1-8, wherein the osmotic pressure comparable to that of human blood has an osmolality of about 280 mOsmol/kg to about 380 mOsmol/kg;
preferably, the liquid preparation has an osmolality of about 280 mOsmol/kg to about 360 mOsmol/kg.

10. The liquid preparation of any one of claims 1-9, wherein the liquid preparation further comprises a surfactant; the surfactant is polysorbate 80, polysorbate 20 or poloxamer 188;
wherein the concentration of the surfactant is about 0.01% w/v to about 0.02% w/v;
preferably, the surfactant is polysorbate 80 at a concentration of about 0.02% w/v.

11. The liquid preparation of any one of claims 1-10, the composition of the liquid preparation is selected from the group consisting of:
(a) anti-PD-Ll humanized monoclonal antibody at 20 mg/ml, 140 mM sodium chloride, 20 mM histidine-histidine hydrochloride, polysorbate 80 at a mass-to-volume ratio of 0.02%, pH 5.8;
(b) anti-PD-Ll humanized monoclonal antibody at 20 mg/ml, 140 mM sodium chloride, 20 mM histidine-histidine hydrochloride, polysorbate 80 at a mass-to-volume ratio of 0.02%, pH 5.0;
(c) anti-PD-Ll humanized monoclonal antibody at 20 mg/ml, 140 mM sodium chloride, 20 mM histidine-histidine acetate, polysorbate 80 at a mass-to-volume ratio of 0.02%, pH 5.8;
(d) anti-PD-Ll humanized monoclonal antibody at 20 mg/ml, 140 mM sodium chloride, 20 mM acetic acid-sodium acetate, polysorbate 80 at a mass-to-volume ratio of 0.02%, pH 5.8;
(e) anti-PDL-1 humanized monoclonal antibody at 20 mg/ml, 140 mM sodium chloride, 20 mM citric acid-sodium citrate, polysorbate 80 at a mass-to-volume ratio of 0.02%, pH 5.8;
(f) anti-PD-Ll humanized monoclonal antibody at 20 mg/ml, 140 mM sodium chloride, 20 mM histidine-histidine hydrochloride, polysorbate 20 at a mass-to-volume ratio of 0.02%, pH 5.8;
(g) anti-PD-Ll humanized monoclonal antibody at 60 mg/ml, 10 mM histidine-histidine hydrochloride, 140 mM sodium chloride, polysorbate 80 at a mass-to-volume ratio of 0.02%, pH 5.7;
(h) anti-PD-Ll humanized monoclonal antibody at 60 mg/ml, 20 mM histidine-histidine hydrochloride, 140 mM sodium chloride, 0.02% w/v polysorbate 80, pH 5.8;
(i) anti-PD-Ll humanized monoclonal antibody at 90 mg/ml, 20 mM histidine-histidine hydrochloride, 140 mM sodium chloride, 0.02% w/v polysorbate 80, pH 5.8;
(j) anti-PD-Ll humanized monoclonal antibody at 120 mg/ml, 20 mM histidine-histidine hydrochloride, 140 mM sodium chloride, 0.02% w/v polysorbate 80, pH 5.8;
(k) anti-PD-Ll humanized monoclonal antibody at 20 mg/ml, 100 mM sodium chloride, 20 mM citric acid-sodium citrate, pH 5.6; and
(l) anti-PD-Ll humanized monoclonal antibody at 20 mg/ml, 20 mM histidine-histidine hydrochloride, 140 mM sodium chloride, 0.02% polysorbate 80, pH 7.0.

12. A product comprising a container filled with the liquid preparation of any one of claims 1-11.

13. The product of claim 12, **characterized in that** the container is a glass vial, a metal alloy container or a pre-filled syringe.

14. Use of the liquid preparation of any one of claims 1-11 or the product of claim 12 in the preparation of a medicament for:
(a) prevention and/or treatment of a tumor or anemia;
(b) adjuvant treatment of a tumor or anemia;
(c) diagnosis of a tumor or anemia;
(d) any combination of (a)-(c).

15. The use according to claim 14, wherein the medicament is administrated separately, in combination, simultaneously, or sequentially with an additional pharmaceutically active agent; wherein the additional pharmaceutically active agent is a chemotherapeutic drug.

16. The use according to claim 14, wherein the tumor is selected from the group consisting of breast cancer, lung cancer such as non-small cell lung cancer or lung squamous cell carcinoma, liver cancer, gastric cancer, bowel cancer such as colon cancer or rectal cancer, esophageal cancer, ovarian cancer, cervical cancer, kidney cancer, prostate cancer, bladder cancer, pancreatic cancer, Merkel cell carcinoma, cholangiocarcinoma, nasopharyngeal carcinoma, head and neck squamous cell carcinoma, glioma, melanoma, leukemia and lymphoma.

17. The use according to claim 16, wherein the lymphoma is Hodgkin's lymphoma or non-Hodgkin's lymphoma; more preferably, the non-Hodgkin's lymphoma is one or more of peripheral T-cell lymphoma, angioimmunoblastic T-cell lymphoma, NK/T-cell lymphoma, and B-cell non-Hodgkin's lymphoma.
